# EUROPEAN PATENT SPECIFICATION

(11) **EP 4 593 934 B1**
(45) Date of publication and mention of the grant of the patent: **09.09.2026**
(21) Application number: 23793030.0
(22) Date of filing: 20.09.2023
(51) Int. Cl.: A61N 1/04, A61N 1/36, A61N 1/40

(54) **COMPOSITIONS AND SYSTEMS FOR REDUCING ELECTROSENSATION AND/OR SKIN IRRITATION**
ZUSAMMENSETZUNGEN UND SYSTEME ZUR REDUZIERUNG VON ELEKTROSENSIERUNG UND/ODER HAUTREIZUNG
COMPOSITIONS ET SYSTÈMES POUR RÉDUIRE L'ÉLECTROSENSATION ET/OU L'IRRITATION DE LA PEAU

(30) Priority: 30.09.2022 US 202263377824 P
(43) Date of publication of application: 06.08.2025
(73) Proprietor: Novocure GmbH, 6340 Baar (CH)
(72) Inventor: GILADI, Moshe, 31905 Haifa (IL); VOLOSHIN-SELA, Tali, 31905 Haifa (IL); AVIGDOR, Lilach, 31905 Haifa (IL)
(74) Representative: Carpmaels & Ransford LLP
(86) International application number: PCT/IB2023/059312
(87) International publication number: WO 2024/069321

(56) References cited:
- WO-A1-2020/077013
- US-A1- 2021 138 233
- US-A1- 2021 220 640
- US-A1- 2022 288 405

## Description

### BACKGROUND

Tumor Treating Fields (TTFields) are low intensity (e.g., 1-3 V/cm) alternating electric fields within the intermediate frequency range (such as, but not limited to, 100-500 kHz) that target solid tumors by disrupting mitosis. This non-invasive treatment targets solid tumors and is described, for example, in US Patent Nos. 7,016,725; 7,089,054; 7,333,852; 7,565,205; 8,244,345; 8,715,203; 8,764,675; 10,188,851; and 10,441,776. TTFields are typically delivered through two pairs of transducer arrays that generate perpendicular fields within the treated tumor; the electrode arrays that make up each of these pairs are positioned on opposite sides of the body part that is being treated. More specifically, for the OPTUNE^{®} system, one pair of electrodes is located to the left and right (LR) of the tumor, and the other pair of electrodes is located anterior and posterior (AP) to the tumor. TTFields are approved for the treatment of glioblastoma multiforme (GBM), and may be delivered, for example, via the OPTUNE^{®} system (Novocure Limited, St. Helier, Jersey), which includes transducer arrays placed on the patient's shaved head.

Alternating electric fields (such as, but not limited to, those at frequencies between 50 kHz and 1 MHz) can also be used to treat medical conditions other than tumors. For example, as described in US Patent No. 10,967,167, alternating electric fields at 50-200 kHz can increase the permeability of the blood brain barrier (BBB) so that, for example, chemotherapy drugs can reach the brain. And as described in U.S. Patent No. 11,103,698, alternating electric fields at 50-500 kHz can increase the permeability of cancer cell membranes so that large molecules can traverse cancer cell membranes.

Each transducer array used for the delivery of TTFields in the OPTUNE^{®} device comprises a set of ceramic disk electrodes, which are coupled to the patient's skin (such as, but not limited to, the patient's shaved head for treatment of GBM) through a layer of conductive medical gel. The purpose of the medical gel is to deform to match the body's contours and to provide good electrical contact between the arrays and the skin; as such, the gel interface bridges the skin and reduces interference. The device is intended to be continuously worn by the patient for 2-4 days before removal for hygienic care and re-shaving (if necessary), followed by reapplication with a new set of arrays.

When treating a subject using alternating electric fields, higher amplitudes are strongly associated with higher efficacy of treatment. However, as the amplitude of the alternating electric field increases, and/or as the frequency of the alternating electric field decreases (e.g., to the vicinity of 100 kHz), some subjects experience an electrosensation effect when the alternating electric field switches direction, particularly at the onset of treatment. This electrosensation could be, for example (but not by way of limitation), a vibratory sensation, paresthesia, and/or a twitching or contraction sensation of muscle fibers.

While not wishing to be bound by any theory, the electrosensation is believed to originate from interactions between the alternating electric fields and nerve cells (i.e., neurons) that are positioned near or adjacent to the transducer arrays. For example, when the current density (e.g., the current through any given electrode divided by the area of that electrode) rises above a threshold value (which may vary from person to person), electrosensation can start to occur and can become more noticeable when the current density is increased further.

Electrosensation can be an impediment to long-term alternating electric field treatment, as the presence of these sensations may discourage some subjects from continuing their treatment using alternating electric fields, particularly at the onset of treatment.

In addition, dermatologic adverse events (dAEs) have been observed with the use of existing medical grade hydrogels with TTField generating systems at an incidence rate of 16% and 22% in the phase III trial and the post-marketing surveillance program, respectively; these dAEs include (but are not limited to) various types of skin irritations, allergic and irritant dermatitis, macerations, mechanical lesions, ulcers, and skin infections. In particular, certain skin irritations can be caused by sweat on the skin. These adverse events are exacerbated by the requirement that the hydrogel remain in continuous contact with the patient's skin for multiple days at a time without an extended period of "breathability" between application of TTField arrays to the skin. (Lacouture et al. (2014) Seminars in Oncology, 41:S1-S14).

Further, when a hydrogel comes into contact with sweat over the wear period, the hydrogel swells and degrades, which increases resistivity. Yet further, loss of the hydrogel interface over the approximately multi-day wear period (such as, but not limited to, by erosion of the adhesiveness and conductivity of the hydrogel) reduces the standard current/electric field generated by the TTFields system and thus decreases the functionality and overall effectiveness of the TTFields treatment.

US 2021/0220640 A1 provides an assembly for use in a TTField - generating system is disclosed that includes at least one electrode in combination with a polymerized conductive hydrogel and a liquid conductive hydrogel disposed on at least a portion of the polymerized hydrogel.

WO 2020/077013 A1 provides an assembly for use in a TTField - generating system that includes at least one electrode in combination with a polymerized conductive hydrogel and a liquid conductive hydrogel disposed on at least a portion of the polymerized hydrogel.

### DETAILED DESCRIPTION

The invention is defined by independent claim 1. Before explaining at least one embodiment of the inventive concept(s) in detail by way of exemplary language and results, it is to be understood that the inventive concept(s) is not limited in its application to the details of construction and the arrangement of the components set forth in the following description. The inventive concept(s) is capable of other embodiments or of being practiced or carried out in various ways. As such, the language used herein is intended to be given the broadest possible scope and meaning; and the embodiments are meant to be exemplary - not exhaustive. Also, it is to be understood that the phraseology and terminology employed herein is for the purpose of description and should not be regarded as limiting.

Unless otherwise defined herein, scientific and technical terms used in connection with the presently disclosed inventive concept(s) shall have the meanings that are commonly understood by those of ordinary skill in the art. Further, unless otherwise required by context, singular terms shall include pluralities and plural terms shall include the singular. The foregoing techniques and procedures are generally performed according to conventional methods well known in the art and as described in various general and more specific references that are cited and discussed throughout the present specification. The nomenclatures utilized in connection with, and the laboratory procedures and techniques of, analytical chemistry, synthetic organic chemistry, and medicinal and pharmaceutical chemistry described herein are those well-known and commonly used in the art. Standard techniques are used for chemical syntheses and chemical analyses.

All patents, published patent applications, and non-patent publications mentioned in the specification are indicative of the level of skill of those skilled in the art to which this presently disclosed inventive concept(s) pertains.

All of the compositions, assemblies, systems, kits, and/or methods disclosed herein can be made and executed without undue experimentation in light of the present disclosure. While the compositions, assemblies, systems, and kits of the inventive concept(s) have been described in terms of particular embodiments, it will be apparent to those of skill in the art that variations may be applied to the compositions without departing from the scope of the inventive concept(s). All such similar substitutions and modifications apparent to those skilled in the art are deemed to be within the scope of the inventive concept(s) as defined by the appended claims.

As utilized in accordance with the present disclosure, the following terms, unless otherwise indicated, shall be understood to have the following meanings:

The use of the term "a" or "an" when used in conjunction with the term "comprising" in the claims and/or the specification may mean "one," but it is also consistent with the meaning of "one or more," "at least one," and "one or more than one." As such, the terms "a," "an," and "the" include plural referents unless the context clearly indicates otherwise. Thus, for example, reference to "a compound" may refer to one or more compounds, two or more compounds, three or more compounds, four or more compounds, or greater numbers of compounds. The term "plurality" refers to "two or more."

The use of the term "at least one" will be understood to include one as well as any quantity more than one, including but not limited to, 2, 3, 4, 5, 10, 15, 20, 30, 40, 50, 100, etc. The term "at least one" may extend up to 100 or 1000 or more, depending on the term to which it is attached; in addition, the quantities of 100/1000 are not to be considered limiting, as higher limits may also produce satisfactory results. In addition, the use of the term "at least one of X, Y, and Z" will be understood to include X alone, Y alone, and Z alone, as well as any combination of X, Y, and Z. The use of ordinal number terminology (i.e., "first," "second," "third," "fourth," etc.) is solely for the purpose of differentiating between two or more items and is not meant to imply any sequence or order or importance to one item over another or any order of addition, for example.

The use of the term "or" in the claims is used to mean an inclusive "and/or" unless explicitly indicated to refer to alternatives only or unless the alternatives are mutually exclusive. For example, a condition "A or B" is satisfied by any of the following: A is true (or present) and B is false (or not present), A is false (or not present) and B is true (or present), and both A and B are true (or present).

As used herein, any reference to "one embodiment," "an embodiment," "some embodiments," "one example," "for example," or "an example" means that a particular element, feature, structure, or characteristic described in connection with the embodiment is included in at least one embodiment. The appearance of the phrase "in some embodiments" or "one example" in various places in the specification is not necessarily all referring to the same embodiment, for example. Further, all references to one or more embodiments or examples are to be construed as non-limiting to the claims.

Throughout this application, the term "about" is used to indicate that a value includes the inherent variation of error for a composition/apparatus/ device, the method being employed to determine the value, or the variation that exists among the study subjects. For example, but not by way of limitation, when the term "about" is utilized, the designated value may vary by plus or minus twenty percent, or fifteen percent, or twelve percent, or eleven percent, or ten percent, or nine percent, or eight percent, or seven percent, or six percent, or five percent, or four percent, or three percent, or two percent, or one percent from the specified value, as such variations are appropriate to perform the disclosed methods and as understood by persons having ordinary skill in the art.

As used in this specification and claim(s), the words "comprising" (and any form of comprising, such as "comprise" and "comprises"), "having" (and any form of having, such as "have" and "has"), "including" (and any form of including, such as "includes" and "include"), or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AAB, BBC, AAABCCCC, CBBAAA, CABABB, and so forth. The skilled artisan will understand that typically there is no limit on the number of items or terms in any combination, unless otherwise apparent from the context.

As used herein, the term "substantially" means that the subsequently described event or circumstance completely occurs or that the subsequently described event or circumstance occurs to a great extent or degree. For example, when associated with a particular event or circumstance, the term "substantially" means that the subsequently described event or circumstance occurs at least 80% of the time, or at least 85% of the time, or at least 90% of the time, or at least 95% of the time. For example, the term "substantially adjacent" may mean that two items are 100% adjacent to one another, or that the two items are within close proximity to one another but not 100% adjacent to one another, or that a portion of one of the two items is not 100% adjacent to the other item but is within close proximity to the other item.

The term "pharmaceutically acceptable" refers to compounds and compositions which are suitable for administration to humans and/or animals without undue adverse side effects such as (but not limited to) toxicity, irritation, and/or allergic response commensurate with a reasonable benefit/risk ratio.

The term "patient" or "subject" as used herein includes human and veterinary subjects. "Mammal" for purposes of treatment refers to any animal classified as a mammal, including (but not limited to) humans, domestic and farm animals, nonhuman primates, and any other animal that has mammary tissue.

The term "treatment" refers to both therapeutic treatment and prophylactic or preventative measures. Those in need of treatment include, but are not limited to, individuals already having a particular condition/disease/infection as well as individuals who are at risk of acquiring a particular condition/disease/infection (e.g., those needing prophylactic/preventative measures). The term "treating" refers to administering an agent/element/method to a patient for therapeutic and/or prophylactic/preventative purposes.

The term "therapeutic composition" or "pharmaceutical composition" as used herein refers to an agent that may be administered *in vivo* to bring about a therapeutic and/or prophylactic/preventative effect.

Administering a therapeutically effective amount or prophylactically effective amount is intended to provide a therapeutic benefit in the treatment, prevention, and/or management of a disease, condition, and/or infection. The specific amount that is therapeutically effective can be readily determined by the ordinary medical practitioner, and can vary depending on factors known in the art, such as (but not limited to) the type of condition/disease/infection, the patient's history and age, the stage of the condition/disease/infection, and the co-administration of other agents.

The term "effective amount" refers to an amount of a biologically active molecule or conjugate or derivative thereof, or an amount of a treatment protocol (i.e., an alternating electric field), sufficient to exhibit a detectable therapeutic effect without undue adverse side effects (such as (but not limited to) toxicity, irritation, and allergic response) commensurate with a reasonable benefit/risk ratio when used in the manner of the inventive concept(s). The therapeutic effect may include, for example but not by way of limitation, preventing, inhibiting, or reducing the occurrence of at least one condition. The effective amount for a subject will depend upon the type of subject, the subject's size and health, the nature and severity of the condition to be treated, the method of administration, the duration of treatment, the nature of concurrent therapy (if any), the specific formulations employed, and the like. Thus, it is not possible to specify an exact effective amount in advance. However, the effective amount for a given situation can be determined by one of ordinary skill in the art using routine experimentation based on the information provided herein.

The terms "administration" and "administering," as used herein, will be understood to include all routes of administration known in the art, including but not limited to, oral, topical, transdermal, parenteral, subcutaneous, intranasal, mucosal, intramuscular, intraperitoneal, and intravenous routes, and including both local and systemic applications. In addition, the compositions of the present disclosure (and/or the methods of administration of same) may be designed to provide delayed, controlled, or sustained release using formulation techniques which are well known in the art.

Turning now to the disclosure, non-claimed methods of reducing, reducing the occurrence of, and/or ameliorating electrosensation in a subject are disclosed, wherein the electrosensation is caused by application of an alternating electric field to the subject. Also disclosed herein are non-claimed methods of reducing, reducing the occurrence of, and/or ameliorating skin irritation in a subject, wherein the skin irritation is caused by application of an alternating electric field to the subject. Yet further, compositions, kits, arrays, and systems related to said methods are also disclosed herein. The compositions, kits, arrays, systems, and methods utilize the application of at least one active agent comprising at least one localized numbing agent and/or at least one anti-hyperhidrosis agent to a portion of the skin of the subject in the target region to which the alternating electric field is applied. The at least one localized numbing agent(s)/anti-hyperhidrosis agent may be present in a separate composition that is applied to the skin or may be associated in some manner with a portion of the system that generates or provides the alternating electric field. In addition, in some non-limiting embodiments, the at least one active agent is both a localized numbing agent and an anti-hyperhidrosis agent.

Certain non-limiting embodiments of the present disclosure are directed to a non-claimed method of reducing electrosensation and/or skin irritation in a subject caused by application of an alternating electric field to the subject. The method includes the administration of at least one composition to at least a portion of a target region of the subject, wherein the at least one composition comprises at least one active agent selected from at least one localized numbing agent, at least one anti-hyperhidrosis agent, and combinations thereof. In some non-limiting embodiments, the at least one active agent is both a localized numbing agent and an anti-hyperhidrosis agent. The method also includes the application of an alternating electric field to the target region of the subject. The alternating electric field may be applied substantially simultaneously or wholly or partially sequentially with the administration of the at least one composition.

Any localized numbing agents known in the art or otherwise contemplated herein may be utilized in accordance with the present disclosure, so long as the localized numbing agent is capable of reducing electrosensation in the target region of the subject to which the alternating electric field is applied. Non-limiting examples of localized numbing agents that may be utilized in accordance with the present disclosure include a *Botulinum* toxin, saxitoxin, tetrodotoxin, lidocaine, bupivacaine, ropivacaine, benzocaine, pramoxine, prilocaine, proparacaine, dibucaine, tetracaine, lignocaine, other sodium channel inhibitors, and the like, as well as any combinations thereof.

In other particular (but non-limiting) embodiments, the at least one localized numbing agent comprises one or more skin numbing agents such as (but not limited to) lidocaine (such as found in skin-numbing creams, gels, sprays, foams, liquids, ointments, and/or injectables like DERMOPLAST^{®} pain relieving spray (Advantice Health, LLC, Cedar Knolls, NJ), LidoRx^{®} controlled release transdermal gel (Gensco Pharma, LLC, Miami, FL), and LIDODERM^{®} hydrogel patch (Teikoku Pharma USA, San Jose, CA); bupivacaine, ropivacaine, and/or lignocaine injectable solutions (or other forms/formulations thereof); benzocaine (such as found in skin-numbing creams, gels, sprays, foams, liquids, ointments, and/or injectables like SOLARCAINE^{®} (Bayer, Boca Raton, FL), LANACANE^{®} (RB Health (US) LLC, Parsippany, NJ), and DERMOPLAST^{®} (Advantice Health, LLC, Cedar Knolls, NJ) products); pramoxine/pramocaine (such as found in skin-numbing creams, gels, sprays, foams, liquids, ointments, and/or injectables like SARNA^{®} Sensitive (Crown Laboratories, Inc., Johnson City, TN), PROCTOFOAM^{®} (Alaven Pharmaceutical LLC, Marietta, GA), and PRAX^{®} (Ferndale Laboratories, Inc., Ferndale, MI) products); dibucaine/cinchocaine (such as found in skin-numbing creams, gels, sprays, foams, liquids, ointments, and/or injectables like NUPERCAINAL^{®} (Dr. Reddy's Laboratories Inc., Princeton, NJ) and Rectacaine products); tetracaine/amethocaine (such as found in skin-numbing creams, gels, sprays, foams, liquids, ointments, and/or injectables like AMETOP^{®} (Alliance Pharmaceutical Limited, Chippenham, UK), PONTOCAINE^{®} (Hospira, Inc., Lake Forest, IL), Viractin, and ZILACTIN^{®} (Blairex Laboratories, Inc., Columbus, IN) products); and the like; as well as any combinations thereof (such as, but not limited to, combinations of lidocaine-bupivacaine, lidocaine-tetracaine, lignocaine-prilocaine, lignocaine-tetracaine, bupivacaine-saxitoxin, etc.).

Any anti-hyperhidrosis agents known in the art or otherwise contemplated herein may be utilized in accordance with the present disclosure, so long as the anti-hyperhidrosis agent is capable of reducing perspiration by the subject's skin in the target region to which the alternating electric field is applied. One non-limiting example of an anti-hyperhidrosis agent that may be utilized in accordance with the present disclosure includes a *Botulinum* toxin.

The active agent(s)-containing composition may be provided in any form/formulation that allows for administration of the composition to the target region of the subject and allows the active agent(s) to penetrate into the subject's skin for reducing the electrosensation and/or skin irritation. Non-limiting examples of forms/formulations that may be utilized include a cream, gel, foam, lotion, ointment, salve, oil, liquid, emulsion, spray, aerosol, dispersion, solid (patch or bandage), an adhesive bandage, and the like, as well as any combinations thereof.

In a particular (but non-limiting) embodiment, the at least one active agent may comprise *Botulinum* toxin complex A (BoNT-A). BoNT-A is currently used to treat numerous medical conditions such as (but not limited to) dystonia, neuromuscular disorders, and pain. In addition, BoNT-A has been approved for use in the treatment of severe axillary hyperhidrosis. The effects of BoNT-A start between two to five days after injection and are maintained between three to six months. BoNT-A exerts its action by preventing the exocytosis of acetylcholine vesicles at the neuromuscular junction, thereby eliciting flaccid paralysis (Caron et al. (2015) PLoS ONE, 10(10): e0140439; Mark Hallett (2015) Toxicon, 107:64-67). In addition, Li et al. (Med Eng Phys. (2017) 43:97-102) examined the immediate impact of BoNT-A injection on the inherent electrical properties of spastic muscles and found that, despite a significant decrease of resistance in the treated muscle, there were no substantial changes in other impedance variables such as reactance, phase angles, or the anisotropy of each of the parameters (i.e., resistance, reactance, and phase angle) after toxin injection.

In addition, the compositions of the present disclosure may contain both at least one localized numbing agent and at least one anti-hyperhidrosis agent. Alternatively, the methods of the present disclosure may include the steps of applying a first composition comprising at least one localized numbing agent and a second composition comprising at least one anti-hyperhidrosis agent. When administered separately, the first composition may be administered simultaneously or wholly or partially sequentially with the second composition. In addition, the use of ordinal terminology is for purposes of identification only; it will be understood that the first composition may be administered before or after the second composition.

The at least one active agent may be present in the composition at any concentration that provides a therapeutically effective concentration of the active agent(s) so as to reduce electrosensation and/or skin irritation upon application of the composition to the skin of the subject. For example, but not by way of limitation, each active agent may be present in the composition at a concentration (wt%) of about 0.001%, about 0.005%, about 0.01%, about 0.02%, about 0.03%, about 0.04%, about 0.05%, about 0.06%, about 0.07%, about 0.08%, about 0.09%, about 0.1%, about 0.2%, about 0.3%, about 0.4%, about 0.5%, about 0.6%, about 0.7%, about 0.8%, about 0.9%, about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, and the like, as well as a range formed from any of the above values (e.g., a range of from about 0.01% to about 20%, etc.), and a range that combines two values that fall between two of the above-referenced values (e.g., a range of from about 0.025% to about 17.5%, etc.).

In particular (but non-limiting) embodiments, the composition comprises *Botulinum* toxin at a concentration in a range of from about 0.1% to about 10%. In particular (but non-limiting) embodiments, the composition comprises lidocaine at a concentration in a range of from about 0.1% to about 10% (and in particular, but not by way of limitation, a range of from about 1% to about 5%). In particular (but non-limiting) embodiments, the composition comprises bupivacaine at a concentration in a range of from about 0.1% to about 1%. In particular (but non-limiting) embodiments, the composition comprises benzocaine at a concentration in a range of from about 1% to about 25% (and in particular, but not by way of limitation, a range of from about 5% to about 20%). In particular (but non-limiting) embodiments, the composition comprises tetracaine at a concentration in a range of from about 0.1% to about 10% (and in particular, but not by way of limitation, a range of from about 0.5% to about 8%). In particular (but non-limiting) embodiments, the composition comprises lignocaine at a concentration in a range of from about 1% to about 15% (and in particular, but not by way of limitation, a range of from about 2% to about 10%). In particular (but non-limiting) embodiments, the composition comprises proparacaine at a concentration in a range of from about 0.1% to about 2%.

However, the concentration of active agent(s) present in the composition may be measured by other mechanisms. For example, in certain non-limiting embodiments, the active agent(s) may be present in the composition at a specific molar concentration. Non-limiting examples of molar concentrations that may be utilized in accordance with the present disclosure include about 0.0001 M, about 0.0005 M, about 0.001 M, about 0.005 M, about 0.01 M, about 0.05 M, about 0.1 M, about 0.2 M, about 0.3 M, about 0.4 M, about 0.5 M, about 0.6 M, about 0.7 M, about 0.8 M, about 0.9 M, about 1 M, about 2 M, about 3 M, about 4 M, about 5 M, or higher, as well as a molar concentration that falls within a range formed of two of the above values (e.g., a range of from about 0.0001 M to about 1 M, a range of from about 0.001 M to about 0.1 M, etc.), or a concentration that falls within a range of two values, each of which falls between two values listed above (e.g.., a range of from about 0.007 M to about 0.86 M, etc.).

In yet another alternative, the active agent(s) may be present in the composition so as to deliver a specific number of units in each dosage. Non-limiting examples of unit dosages that may be utilized in accordance with the present disclosure include about 0.1, about 0.2, about 0.3, about 0.4, about 0.5, about 0.6, about 0.7, about 0.8, about 0.9, about 1, about 2, about 3, about 4, about 5, about 6, about 7, about 8, about 9, about 10, about 15, about 20, about 25, about 30, about 35, about 40, about 45, about 50, about 55, about 60, about 65, about 70, about 75, about 80, about 85, about 90, about 95, about 100, about 125, about 150, about 175, or about 200 units, or higher, as well as a unit dosage range formed of two of the above values (e.g., a range of from about 0.5 units to about 100 units, etc.), or a unit dosage range formed of two values, each of which falls between two values listed above (e.g., a range of from about 0.25 units to about 180 units, etc.).

The compositions may be formulated for repeated administration to the patient, based upon the effective duration of the active agent(s). Dosages may be repeated as needed, for example (but not by way of limitation), about once every hour, about once every two hours, about once every three hours, about once every four hours, about once every five hours, about once every six hours, about once every seven hours, about once every eight hours, about once every nine hours, about once every 10 hours, about once every 11 hours, about once every 12 hours, about once a day, about once every two days, about once every three days, about once every four days, about once every five days, about once every six days, about once per week, about once every two weeks, about once every three weeks, etc., as well as a range formed from two of the above values.

In certain non-limiting embodiments, the compositions of the present disclosure may be designed to provide modified release (such as, but not limited to, extended, controlled, and/or sustained release) of one or more of the active agent(s) using formulation techniques which are well known in the art. Non-limiting examples of formulation techniques include coatings, polymer conjugation, the use of one or more delivery systems in which the localized numbing agent(s) is disposed or encapsulated (such as, but not limited to, liposomes, lipid-based particles, microspheres, microparticles, nanoparticles, polymer conjugates, a SABER (sucrose acetate isobutyrate) system, collagen implants, DepoFoam, and combinations thereof), and the like, as well as combinations thereof. The various formulation methods for providing modified release of an active agent are well known in the art, and therefore no further description thereof is deemed necessary.

The use of modified release formulations may allow the one or more active agent(s) to be released from the composition over an extended period of time, such as (but not limited to) at least about eight hours, at least about 12 hours, at least about 18 hours, at least about one day, at least about two days, at least about three days, at least about four days, at least about five days, at least about six days, at least about seven days (i.e., at least about one week), at least about eight days, at least about nine days, at least about 10 days, at least about 11 days, at least about 12 days, at least about 13 days, at least about 14 days (i.e., at least about two weeks), at least about 15 days, at least about 16 days, at least about 17 days, at least about 18 days, at least about 19 days, at least about 20 days, at least about 21 days (i.e., at least about three weeks), at least about 22 days, at least about 23 days, at least about 24 days, at least about 25 days, at least about 26 days, at least about 27 days, at least about 28 days, at least about 29 days, at least about 30 days, at least about one month, and the like, as well as a range formed from any of the above values (i.e., a range of from about one day to about two weeks, a range of from about two weeks to about three weeks, etc.).

Specific non-limiting examples of modified release formulations of localized numbing agent(s) that may be utilized in accordance with the present disclosure include a liposomal formulation of saxitoxin (saxitoxin plus dexamethasone liposomes, which has been shown to provide a nerve blockade for up to 7.5 days (Epstein-Barash et al. (2009) PNAS, 106(17):7125-7130)); polymer-tetrodotoxin conjugates (which have been shown to produce a range of durations of nerve block from several hours to three days (Elofsson et al. (2019) Nature Commun, 10:2566)); tetrodotoxin microspheres (Kohane et al. (2003) Pain, 104:415-421); a liposomal formulation of bupivacaine (EXPAREL^{®} (bupivacaine liposomal injectable suspension in DEPOFOAM^{®}), which is FDA approved and provides up to 48-72 hours of pain relief)); SABER-bupivacaine (SABER was developed as a bioerodable injectable depot system with the potential for delivering a drug over a period of days to months); bupivacaine-collagen matrix/implant (XARACOLL^{®} implant (Innocoll Pharmaceuticals Limited, Princeton, NJ), shown to provide analgesia for 24 - 72 hours); and the like; as well as any combinations thereof.

The compositions of the present disclosure may be administered via any routes of administration that allow for localized numbing of at least a portion of the skin in the target region of the subject. Selected routes of administration include (but are not limited to) topical, transdermal, intravenous, intramuscular, intradermal, intraperitoneal, subcutaneous, spinal, or other parenteral routes of administration, for example by injection or infusion.

In certain particular (but non-limiting) embodiments, microneedling is utilized in the administration of the at least one composition, wherein the active agent(s)-containing composition is coated upon microneedles prior to administration to the subject. For example, but not by way of limitation, the use of microneedling to deliver *Botulinum* toxin to treat various cosmetic concerns is currently utilized in the art.

The at least one active agent may be disposed and administered in any formulation known in the art or otherwise contemplated herein that will reduce electrosensation and/or skin irritation caused by application of the alternating electric fields to the subject. For example, but not by way of limitation, the at least one active agent-containing composition may be administered in the form of a pharmaceutical composition that comprises the at least one active agent in combination with at least one pharmaceutically-acceptable carrier. Non-limiting examples of suitable pharmaceutically acceptable carriers that may be utilized in accordance with the present disclosure include water; saline; dextrose solutions; fructose or mannitol; calcium carbonate; cellulose; ethanol; oils of animal, vegetative, or synthetic origin; carbohydrates, such as glucose, sucrose, or dextrans; antioxidants, such as ascorbic acid or glutathione; chelating agents; low molecular weight proteins; detergents; liposomal carriers; buffered solutions, such as sodium chloride, saline, phosphate-buffered saline, and/or other substances which are physiologically acceptable and/or safe for use; diluents; excipients such as polyethylene glycol (PEG); or any combination thereof. Suitable pharmaceutically acceptable carriers for pharmaceutical formulations are described, for example, in Remington: The Science and Practice of Pharmacy, 23rd ed (2020).

In addition, the composition containing the at least one active agent may contain other agents that allow for administration of the compositions via a particular administration route. For example, but not by way of limitation, the compositions may be formulated for administration by topical, transdermal, and/or subcutaneous routes, or by any other route described or otherwise contemplated herein. Based on the route of administration, the compositions may also contain one or more additional components in addition to the active agent (i.e., localized numbing agent and/or anti-hyperhidrosis agent). Examples of additional secondary compounds that may be present include, but are not limited to, fillers, salts, buffers, preservatives, stabilizers, solubilizers, wetting agents, emulsifying agents, dispersing agents, and other materials well known in the art.

Further, in certain particular (but non-limiting) embodiments, the composition containing the at least one localized numbing agent and/or at least one anti-hyperhidrosis agent may further contain other active agents that are typically combined with the numbing/anti-hyperhidrosis agent(s) in existing formulations. For example (but not by way of limitation), certain topical numbing creams include hydrocortisone to reduce redness, itching, and swelling. In addition, certain topical numbing creams include epinephrine, norepinephrine, or phenylephrine to improve the depth and duration of the anesthesia at the target site.

In certain non-limiting embodiments, the composition containing the at least one active agent may be applied prior to application of the electrodes, but may also be applied substantially simultaneously with and/or following application of the electrodes.

In addition, the composition containing the at least one localized numbing agent and/or at least one anti-hyperhidrosis agent may be applied prior to application of the alternating electric field, but may also be applied substantially simultaneously with application of the alternating electric field. For example (but not by way of limitation), the composition may be administered before the application of the alternating electric field has commenced by a period of at least about 1 minute, about 5 minutes, about 10 minutes, about 15 minutes, about 30 minutes, about 45 minutes, about 1 hour, about 1.5 hours, about 2 hours, about 3 hours, about 4 hours, about 5 hours, about 6 hours, about 7 hours, about 8 hours, about 9 hours, about 10 hours, about 12 hours, about 15 hours, about 18 hours, about 21 hours, about 24 hours, about 27 hours, about 30 hours, about 33 hours, about 36 hours, about 39 hours, about 42 hours, about 45 hours, about 48 hours, about 51 hours, about 54 hours, about 57 hours, about 60 hours, about 63 hours, about 66 hours, about 69 hours, about 72 hours, about 75 hours, about 78 hours, about 81 hours, about 84 hours, about 87 hours, about 90 hours, about 93 hours, about 96 hours, and the like, as well as a range formed from any of the above values (i.e., a range of from about 1 minute to about 24 hours, etc.), and a range that combines two integers that fall between two of the above-referenced values (i.e., a range of from about 14 minutes to about 94 hours, etc.).

Also, when two or more compositions are administered, each composition may be independently applied prior to application of the alternating electric field and/or substantially simultaneously with application of the alternating electric field.

The alternating electric fields applied in accordance with the non-claimed methods of the present disclosure may be utilized for any therapeutic purposes disclosed or otherwise contemplated herein. In one non-limiting embodiment, the alternating electric fields are applied to treat any types of cancer cells/cancers/tumors that respond to TTField treatment. Non-limiting examples cancer cells/cancers/tumors that can be treated in accordance with the present disclosure include hepatocellular carcinomas, glioblastomas, pleural mesotheliomas, differentiated thyroid cancers, advanced renal cell carcinomas, ovarian cancers, breast cancers, pancreatic cancers, lung cancers (such as, but not limited to, non-small cell lung cancers), and the like, as well as any combination thereof. In another non-limiting embodiment, the alternating electric fields are applied to increase the permeability of tight junctions between endothelial cells, for example in the blood brain barrier or in the intestine.

Any type of conductive or non-conductive electrode(s) and/or transducer array(s) that can be utilized for generating an alternating electric field that are known in the art or otherwise contemplated herein may be utilized for generation of the alternating electric field in accordance with the methods of the present disclosure. Non-limiting examples of electrodes and transducer arrays that can be utilized for generating an alternating electric field in accordance with the present disclosure include those that function as part of a TTField system as described, for example but not by way of limitation, in US Patent Nos. 7,016,725; 7,089,054; 7,333,852; 7,565,205; 8,244,345; 8,715,203; 8,764,675; 10,188,851; and 10,441,776; and in US Patent Application Nos. US 2018/0160933; US 2019/0117956; US 2019/0307781; and US 2019/0308016.

The alternating electric field may be generated at any frequency in accordance with the present disclosure. For example (but not by way of limitation), the alternating electric field may have a frequency of about 50 kHz, about 75 kHz, about 100 kHz, about 125 kHz, about 150 kHz, about 175 kHz, about 200 kHz, about 225 kHz, about 250 kHz, about 275 kHz, about 300 kHz, about 325 kHz, about 350 kHz, about 375 kHz, about 400 kHz, about 425 kHz, about 450 kHz, about 475 kHz, about 500 kHz, about 550 kHz, about 600 kHz, about 650 kHz, about 700 kHz, about 750 kHz, about 800 kHz, about 850 kHz, about 900 kHz, about 950 kHz, about 1 MHz, about 2 MHz, about 3 MHz, about 4 MHz, about 5 MHz, about 6 MHz, about 7 MHz, about 8 MHz, about 9 MHz, about 10 MHz, and the like, as well as a range formed from any of the above values (e.g., a range of from about 50 kHz to about 10 MHz, a range of from about 50 kHz to about 1 MHz, a range of from about 100 kHz to about 500 kHz, a range of from about 150 kHz to about 300 kHz, etc.), and a range that combines two integers that fall between two of the above-referenced values (e.g., a range of from about 122 kHz to about 313 kHz, a range of from about 78 kHz to about 298 kHz, etc.).

In certain particular (but non-limiting) embodiments, the alternating electric field may be imposed at two or more different frequencies. When two or more frequencies are present, each frequency is selected from any of the above-referenced values, or a range formed from any of the above-referenced values, or a range that combines two integers that fall between two of the above-referenced values.

The alternating electric field may have any field strength in the subject/cells, so long as the alternating electric field is capable of functioning in accordance with the present disclosure. For example (but not by way of limitation), the alternating electric field may have a field strength of at least about 1 V/cm, about 1.5 V/cm, about 2 V/cm, about 2.5 V/cm, about 3 V/cm, about 3.5 V/cm, about 4 V/cm, about 4.5 V/cm, about 5 V/cm, about 5.5 V/cm, about 6 V/cm, about 6.5 V/cm, about 7 V/cm, about 7.5 V/cm, about 8 V/cm, about 9 V/cm, about 9.5 V/cm, about 10 V/cm, about 10.5 V/cm, about 11 V/cm, about 11.5 V/cm, about 12 V/cm, about 12.5 V/cm, about 13 V/cm, about 13.5 V/cm, about 14 V/cm, about 14.5 V/cm, about 15 V/cm, about 15.5 V/cm, about 16 V/cm, about 16.5 V/cm, about 17 V/cm, about 17.5 V/cm, about 18 V/cm, about 18.5 V/cm, about 19 V/cm, about 19.5 V/cm, about 20 V/cm, and the like, as well as a range formed from any of the above values (e.g., a range of from about 1 V/cm to about 20 V/cm, a range of from about 1 V/cm to about 10 V/cm, a range of from about 1 V/cm to about 4 V/cm, etc.), and a range that combines two integers that fall between two of the above-referenced values (e.g., a range of from about 1.1 V/cm to about 18.6 V/cm, a range of from about 1.2 V/cm to about 9.8 V/cm, a range of from about 1.3 V/cm to about 4.7 V/cm, etc.).

The alternating electric field may be applied for any period of time sufficient to achieve a therapeutic purpose, such as, but not limited to, a reduction in viability of cancer cells and/or a reduction in tumor volume (and/or a prevention of increase in tumor volume). For example, but not by way of limitation, the alternating electric field may be applied for at least about 1 hour, about 2 hours, about 3 hours, about 4 hours, about 5 hours, about 6 hours, about 7 hours, about 8 hours, about 9 hours, about 10 hours, about 11 hours, about 12 hours, about 15 hours, about 18 hours, about 21 hours, about 24 hours, about 27 hours, about 30 hours, about 33 hours, about 36 hours, about 39 hours, about 42 hours, about 45 hours, about 48 hours, about 51 hours, about 54 hours, about 57 hours, about 60 hours, about 63 hours, about 66 hours, about 69 hours, about 72 hours, about 75 hours, about 78 hours, about 81 hours, about 84 hours, about 87 hours, about 90 hours, about 93 hours, about 96 hours, and the like, as well as a range formed from any of the above values (e.g., a range of from about 24 hours to about 72 hours, etc.), and a range that combines two integers that fall between two of the above-referenced values (e.g., a range of from about 14 hours to about 68 hours, etc.).

In a particular (but non-limiting) embodiment, the period of time that the alternating electric field is applied is at least about 24 hours.

In certain particular (but non-limiting) embodiments, the non-claimed method includes one or more additional steps. For example (but not by way of limitation), the method may further include repeating any of the steps one or more times. Each of the steps can be repeated as many times as necessary. When application of the alternating electric field is repeated, the transducer arrays may be placed in slightly different positions on the subject than their original placement; relocation of the arrays in this manner may further aid in treatment of the disease/condition/tumor/cancer.

In addition, any of the steps of administering the active agent(s)-containing composition may be repeated at various times and at various intervals to follow any known and/or generally accepted dosage regimen for the localized numbing agent(s)/anti-hyperhidrosis agent(s). For example (but not by way of limitation), the composition may be administered one or more times per day (or one or more times over a period of multiple days) over the course of an extended period of time (such as, but not limited to, at least about one week, about two weeks, about three weeks, about four weeks, about one month, about five weeks, about six weeks, about seven weeks, about eight weeks, about two months, etc.). In addition, when application of the alternating electric field is repeated and the transducer arrays are changed during the repeating steps, the administration of the active agent(s)-containing composition may be repeated each time the transducer arrays are changed. Also, when the transducer arrays are placed in slightly different positions on the subject than their original placement during the repeating steps, the active agent(s)-containing composition may be administered in multiple steps to the different sites to which the transducer arrays are subsequently adhered.

For example, in particular (but non-limiting) embodiments, the non-claimed method is further defined as including the steps of: (1) administering at least one composition to at least a portion of a first target region of the subject, wherein the at least one composition comprises at least one localized numbing agent and/or at least one anti-hyperhidrosis agent; (2a) applying a first alternating electric field-generating system transducer array to the first target region of the subject; (2) applying an alternating electric field to the first target region of the subject using the first transducer array; (3) removing the first transducer array from the subject; (4) administering the at least one composition comprising at least one localized numbing agent and/or at least one anti-hyperhidrosis agent to a second target region of the subject; (5) applying a second alternating electric field-generating system transducer array to the second target region of the subject; and (6) applying an alternating electric field to the second target region of the subject using the second transducer array. In a particular (but non-limiting) embodiment, the first and second target regions are the same. In another particular (but non-limiting) embodiment, the first and second target regions are different.

Electrosensation is typically observed for a relatively short period after beginning treatment, such as during an initial treatment period for the first two months, or first six weeks, or first one month, or first four weeks, or first three weeks, or first two weeks, or first week, etc. After that initial period, patients typically become acclimated to the treatment and more tolerant of any electrosensation present, and as such, compliance issues decrease with extended alternating electric field treatment. Therefore, the methods of the present disclosure include use of the localized numbing agent(s)/anti-hyperhidrosis agent(s)-containing composition for an extended period of time as well as use of the localized numbing agent(s)/anti-hyperhidrosis agent(s)-containing composition for a limited period of time (such as, but not limited to, about two months, about eight weeks, about seven weeks, about six weeks, about five weeks, about one month, about four weeks, about three weeks, about two weeks, about one week, or less), even though the alternating electric field treatment may be utilized for a longer period of time. In addition, the numbing and/or reduced perspiration provided by the active agent(s)-containing composition may be achieved by application of multiple doses of composition over a period of time, or by application of a single dose of a controlled/extended-release formulation of the composition.

In a particular (but non-limiting) embodiment, the composition comprising the at least one localized numbing agent and/or at least one anti-hyperhidrosis agent provides effective relief from electrosensation/skin irritation for an extended period in a range of from about two to about four days (or longer), and this extended period is about the same as or longer than a period in which the transducer arrays are applied to the skin of the patient. Therefore, in this non-limiting embodiment, the composition containing the at least one active agent can be reapplied each time that the transducer arrays are changed.

As stated herein above, the composition containing the at least one localized numbing agent and/or at least one anti-hyperhidrosis agent may be administered to the subject separately in a topical/transdermal/injectable formulation. Alternatively, the composition may be added to, included, or otherwise associated with one or more components of the alternating electric field-generating system (such as, but not limited to, a hydrogel, an adhesive for attaching the electrodes, disposed in the space between electrodes, etc.). In this manner, the composition containing the at least one localized numbing agent and/or at least one anti-hyperhidrosis agent may be applied directly between the subject's skin and the electrodes, or the composition may be contained within a material around the electrodes, etc. When the active agent(s)-containing composition is associated with a component of the alternating electric field-generating system, then steps (1) and (2) of the method are performed substantially simultaneously.

Certain non-limiting embodiments of the present disclosure include components of the alternating electric field-generating system having any of the active agent(s)-containing compositions associated therewith. For example (but not by way of limitation), the present disclosure includes a polymerized conductive hydrogel for application to a patient's skin and for placement between the patient's skin and at least one transducer array that generates an alternating electric field having a frequency in a range of from about 50 kHz to about 10 MHz; in addition, the polymerized conductive hydrogel comprises at least one localized numbing agent and/or at least one anti-hyperhidrosis agent disposed therein or attached thereto, wherein said agent(s) includes any of the numbing/anti-hyperhidrosis agents described or otherwise contemplated herein, and in any of the formulations described or otherwise contemplated herein.

The polymerized conductive hydrogel may be in any form that allows the assembly to function in accordance with the present disclosure. In certain particular (but non-limiting) embodiments, the hydrogel is sterile. In addition, in certain non-limiting embodiments, the hydrogel will not substantially degrade upon exposure to sterilization conditions that include gamma rays or ethylene oxide gas. In certain particular (but non-limiting) embodiments, the polymerized hydrogel is semi-solid.

The polymerized hydrogel may be formed of any hydrophilic polymer that allows the hydrogel to function in accordance with the present disclosure. For example (but not by way of limitation), the hydrogel may be a polyacrylic acid gel, a povidone gel, or a cellulose gel. In addition, the hydrogel may comprise at least one of chitosan, alginate, agarose, methylcellulose, hyaluronan, collagen, laminin, matrigel, fibronectin, vitronectin, poly-1-lysine, proteoglycans, fibrin glue, gels made by decellularization of engineered and/or natural tissues, as well as any combinations thereof. Further, the gel may comprise at least one of polyglycolic acid (PGA), polylactic acid (PLA), poly-caprolactone (PCL), polyvinyl alcohol (PVA), polyethylene glycol (PEG), methyl methacrylate, poly(methyl methacrylate) (PMMA), poly(2-hydroxyethyl methacrylate) (PolyHEMA), poly(glycerol sebacate), polyurethanes, poly(isopropylacrylamide), poly(N-isopropylacrylamide), or any combination thereof.

The polymerized hydrogel may be provided with any pH that does not damage the skin of a patient or cause chemical irritation of the skin upon prolonged exposure to the gel. For example (but not by way of limitation), the gel may have a pH of about 6, about 6.5, about 7, about 7.5, about 8, as well as a range formed from any of the above values (i.e., a range of from about 6 to about 8, a range of from about 6.5 to about 7.5, etc.).

The polymerized hydrogel may be provided with any thickness that allows the gel to function in accordance with the present disclosure. Non-limiting examples of thicknesses that may be utilized in accordance with the present disclosure include about 1 mil, about 5 mil, about 10 mil, about 15 mil, about 20 mil, about 25 mil, about 30 mil, about 35 mil, about 40 mil, about 45 mil, about 50 mil, about 55 mil, about 60 mil, about 65 mil, about 70 mil, about 75 mil, about 80 mil, about 85 mil, about 90 mil, about 95 mil, about 100 mil, or higher, as well as a range that combines any two of the above-referenced values (i.e., a range of from about 10 mil to about 50 mil, etc.), and a range that combines two integers that fall between two of the above-referenced values (i.e., a range of from about 12 mil to about 48 mil, etc.).

The polymerized hydrogel may be provided with any skin adhesion rate that allows the gel to function in accordance with the present disclosure. For example (but not by way of limitation), the skin adhesion rate of the gel may be at least about 100 g/inch, at least about 110 g/inch, at least about 120 g/inch, at least about 130 g/inch, at least about 140 g/inch, at least about 150 g/inch, at least about 160 g/inch, at least about 170 g/inch, at least about 180 g/inch, at least about 190 g/inch, at least about 200 g/inch, at least about 210 g/inch, at least about 220 g/inch, at least about 230 g/inch, at least about 240 g/inch, at least about 250 g/inch, at least about 260 g/inch, at least about 270 g/inch, at least about 280 g/inch, at least about 290 g/inch, at least about 300 g/inch, or higher, as well as a range of any of the above values (a range of from about 120 g/inch to about 300 g/inch, etc.), and a range that combines two integers that fall between two of the above-referenced values (i.e., a range of from about 115 g/inch to about 295 g/inch, etc.).

Another component of the alternating electric field-generating system with which the localized numbing/anti-hyperhidrosis agent(s)-containing composition may be associated therewith is the transducer arrays. Therefore, certain non-limiting embodiments of the present disclosure are directed to an alternating electric field-generating system transducer array that includes at least two insulated electrodes, wherein each of the electrodes generates an alternating electric field having a frequency in a range from about 50 kHz to about 10 MHz (e.g., from about 50 kHz to about 1 MHz, about 50 kHz to about 500 kHz, or about 100 kHz to about 300 kHz). The transducer array also includes at least one composition associated therewith, wherein the at least one composition comprises at least one of any of the localized numbing agents/anti-hyperhidrosis agents disclosed or otherwise contemplated herein and in any of the formulations disclosed or otherwise contemplated herein.

In certain non-limiting embodiments, the active agent(s)-containing composition is disposed on or between the at least two electrodes. Alternatively, the active agent(s)-containing composition may be disposed in at least one adhesive for adhering the transducer array to a skin of a patient. In yet another non-limiting alternative, the system further includes at least one bandage for attaching the transducer array to a skin of a patient, and the at least one active agent(s)-containing composition is incorporated within at least a portion of the at least one bandage and/or disposed on at least a portion of a lower surface of the at least one bandage.

In certain particular (but non-limiting) embodiments, each electrode may be an insulated electrode that comprises at least one non-conducting layer, at least one conducting layer, and a high capacitance layer having a top surface and a bottom surface; in addition, at least one opening may be disposed between the top surface and the bottom surface of the high capacitance layer.

Certain non-limiting embodiments of the present disclosure are related to kits that include any of the components of the alternating electric field-generating systems (such as, but not limited to, one or more transducer arrays and/or one or more hydrogel compositions, as disclosed in US Patent Nos. 7,016,725; 7,089,054; 7,333,852; 7,565,205; 8,244,345; 8,715,203; 8,764,675; 10,188,851; and 10,441,776; and in US Patent Application Nos. US 2018/0160933; US 2019/0117956; US 2019/0307781; and US 2019/0308016) in combination with any of the compositions containing at least one localized numbing agent and/or at least one anti-hyperhidrosis agent as disclosed or otherwise contemplated herein and in any of the formulations disclosed or otherwise contemplated herein. The kits may optionally further include one or more of any of the optional compositions disclosed or otherwise contemplated herein. The kits may optionally further include one or more devices (or one or more components of devices) utilized in one or more additional therapy steps.

In certain particular (but non-limiting) embodiments, the kit may further include one or more elements or devices for administering the composition containing at least one localized numbing agent and/or at least one anti-hyperhidrosis agent to the subject. For example (but not by way of limitation), when the localized numbing/anti-hyperhidrosis agent(s)-containing composition is delivered via microneedling, the kit may further include at least one assembly having a plurality of microneedles. The composition comprising at least one localized numbing agent and/or at least one anti-hyperhidrosis agent may be present in the kit separate from the assembly (or other delivery element/device); alternatively, at least a portion of the microneedles (or other delivery element/device) may be coated with the localized numbing/anti-hyperhidrosis agent(s)-containing composition. In a particular (but non-limiting) embodiment, the at least one composition comprising at least one localized numbing agent and/or at least one anti-hyperhidrosis agent is associated with a distal portion of at least one of the plurality of microneedles.

In a particular (but non-limiting) embodiment, the kit may further include instructions for performing any of the methods disclosed or otherwise contemplated herein. For example (but not by way of limitation), the kit may include instructions for applying the composition comprising at least one localized numbing agent and/or at least one anti-hyperhidrosis agent to the skin of the patient, instructions for applying one or more components of the alternating electric field to the skin of the patient, instructions for applying the alternating electric field to the patient, optionally instructions for when and how to reapply the composition comprising at least active agent and/or the one or more components of the alternating electric field to the skin of the patient, and/or instructions for when to activate and turn off the alternating electric field.

In addition to the components described in detail herein above, the kits may further contain other component(s)/reagent(s) for performing any of the particular methods described or otherwise contemplated herein. For example (but not by way of limitation), the kits may additionally include: (i) components for preparing the skin prior to disposal of the hydrogel compositions and/or transducer arrays thereon (i.e., a razor, a cleansing composition or wipe/towel, etc.); (ii) components for removal of the gel/transducer array(s); (iii) components for cleansing of the skin after removal of the gel/transducer array(s); and/pr (iv) other components utilized with the system (i.e., conductive material, nonconductive material, a soothing gel or cream, a bandage, etc.). The nature of these additional component(s)/reagent(s) will depend upon the particular treatment format, and identification thereof is well within the skill of one of ordinary skill in the art; therefore, no further description thereof is deemed necessary. Also, the components/reagents present in the kits may each be in separate containers/compartments, or various components/reagents can be combined in one or more containers/compartments, depending on the sterility, cross-reactivity, and stability of the components/reagents.

The kit may be disposed in any packaging that allows the components present therein to function in accordance with the present disclosure. In certain non-limiting embodiments, the kit further comprises a sealed packaging in which the components are disposed. In certain particular (but non-limiting) embodiments, the sealed packaging is substantially impermeable to air and/or substantially impermeable to light.

In addition, the kit can further include a set of written instructions explaining how to use one or more components of the kit. A kit of this nature can be used in any of the methods described or otherwise contemplated herein.

In certain non-limiting embodiments, the kit has a shelf life of at least about six months, such as (but not limited to), at least about nine months, or at least about 12 months.

Certain non-limiting embodiments of the present disclosure are related to systems that include any of the components of the alternating electric field-generating systems (such as, but not limited to, one or more transducer arrays and/or one or more hydrogel compositions, as disclosed in US Patent Nos. 7,016,725; 7,089,054; 7,333,852; 7,565,205; 8,244,345; 8,715,203; 8,764,675; 10,188,851; and 10,441,776; and in US Patent Application Nos. US 2018/0160933; US 2019/0117956; US 2019/0307781; and US 2019/0308016) in combination with at least one of any of the compositions comprising localized numbing agent(s) and/or anti-hyperhidrosis agent(s) disclosed or otherwise contemplated herein and in any of the formulations disclosed or otherwise contemplated herein. The systems may optionally further include one or more of any of the optional compositions disclosed or otherwise contemplated herein.

Certain non-limiting embodiments of the present disclosure are directed to an assembly comprising a plurality of microneedles, wherein at least a portion of the plurality of microneedles has at least one composition associated therewith, wherein the at least one composition comprises at least one of any of the localized numbing/anti-hyperhidrosis agents disclosed or otherwise contemplated herein and in any of the formulations disclosed or otherwise contemplated herein. In particular (but non-limiting) embodiments, at least a portion of the microneedles are coated with the localized numbing/anti-hyperhidrosis agent(s)-containing composition. In a particular (but non-limiting) embodiment, the at least one composition comprising at least one localized numbing agent and/or at least one anti-hyperhidrosis agent is associated with a distal portion of at least one of the plurality of microneedles.

While the above disclosures describe the inventive concept(s) in conjunction with the specific experimentation, results, and language set forth, it is evident that many alternatives, modifications, and variations will be apparent to those skilled in the art. Accordingly, it is intended to embrace all such alternatives, modifications, and variations that fall within the broad scope of the present disclosure.

## Claims

1. A hydrogel, comprising:
a polymerized conductive hydrogel for application to a patient's skin and for placement between the patient's skin and at least one transducer array that generates an alternating electric field having a frequency in a range of from about 50 kHz to about 10 MHz, wherein the polymerized conductive hydrogel comprises at least one composition disposed therein or attached thereto, wherein the at least one composition comprises at least one active agent selected from at least one localized numbing agent, at least one anti-hyperhidrosis agent, and combinations thereof; wherein:
the at least one composition is formulated for controlled or extended release and comprises at least one delivery system for delivery of the at least one active agent, wherein the delivery system is selected from the group consisting of liposomes, lipid-based particles, microspheres, microparticles, nanoparticles, polymer conjugates, a sucrose acetate isobutyrate system, collagen implants, and combinations thereof.

2. The hydrogel of claim 1, wherein the hydrogel is sterile.

3. The hydrogel of claim 1 or claim 2, wherein the hydrogel is a polyacrylic acid gel, a povidone gel, or a cellulose gel.

4. The hydrogel of any preceding claim, wherein the hydrogel comprises at least one additional substance selected from the group consisting of chitosan, alginate, agarose, methylcellulose, hyaluronan, collagen, laminin, matrigel, fibronectin, vitronectin, poly-1-lysine, proteoglycans, fibrin glue, gels made by decellularization of engineered and natural tissues, and combinations thereof.

5. The hydrogel of any preceding claim, wherein the hydrogel comprises at least one polymer selected from the group consisting of polyglycolic acid (PGA), polylactic acid (PLA), poly-caprolactone (PCL), polyvinyl alcohol (PVA), polyethylene glycol (PEG), methyl methacrylate, poly(methyl methacrylate) (PMMA), poly(2-hydroxyethyl methacrylate) (PolyHEMA), poly(glycerol sebacate), polyurethanes, poly(isopropylacrylamide), poly(N-isopropylacrylamide), and combinations thereof.

6. The hydrogel of any preceding claim, wherein the hydrogel has a pH in a range of from about 6.5 to about 7.5.

7. The hydrogel of any preceding claim, wherein the skin adhesion of the hydrogel is at least about 120 g/inch.

8. The hydrogel of any preceding claim, wherein the hydrogel has a thickness in a range of from about 10 mil to about 50 mil.

9. The hydrogel of any preceding claim wherein, the hydrogel has a shelf life of at least about six months.

10. The hydrogel of any preceding claim, wherein the at least one localized numbing agent comprises at least one substance selected from the group consisting of a *Botulinum* toxin, saxitoxin, tetrodotoxin, lidocaine, bupivacaine, ropivacaine, benzocaine, pramoxine, prilocaine, proparacaine, dibucaine, tetracaine, lignocaine, and combinations thereof.

11. The hydrogel of any preceding claim wherein the at least one anti-hyperhidrosis agent comprises at least one *Botulinum* toxin, and combinations thereof.

12. The hydrogel of claim 10 or claim 11 wherein the *Botulinum* toxin is *Botulinum* toxin complex A.

13. An alternating electric field-generating system transducer array, comprising:
at least two insulated electrodes, wherein each of the electrodes generates an alternating electric field having a frequency in a range from about 50 kHz to about 10 MHz; and
the hydrogel of any preceding claim.

## Patentansprüche

1. Hydrogel, umfassend:
ein polymerisiertes leitfähiges Hydrogel zur Anwendung auf einer Haut eines Patienten und zur Platzierung zwischen der Haut des Patienten und mindestens einem Wandlerarray, das ein elektrisches Wechselfeld erzeugt, das eine Frequenz im Bereich von etwa 50 kHz bis etwa 10 MHz aufweist, wobei das polymerisierte leitfähige Hydrogel mindestens eine darin angeordnete oder daran befestigte Zusammensetzung umfasst, wobei die mindestens eine Zusammensetzung mindestens einen Wirkstoff umfasst, der aus mindestens einem lokalen Betäubungsmittel, mindestens einem Mittel gegen Hyperhidrose und Kombinationen davon ausgewählt ist; wobei
die mindestens eine Zusammensetzung für eine kontrollierte oder verlängerte Freisetzung formuliert ist und mindestens ein Abgabesystem zum Abgeben des mindestens einen Wirkstoffs umfasst, wobei das Abgabesystem aus der Gruppe ausgewählt ist, bestehend aus Liposomen, lipidbasierten Partikeln, Mikrosphären, Mikropartikeln, Nanopartikeln, Polymerkonjugaten, Kollagenimplantaten und Kombinationen davon.

2. Hydrogel nach Anspruch 1, wobei das Hydrogel steril ist.

3. Hydrogel nach Anspruch 1 oder Anspruch 2, wobei das Hydrogel ein Polyacrylsäuregel, ein Povidongel oder ein Cellulosegel ist.

4. Hydrogel nach einem vorstehenden Anspruch, wobei das Hydrogel mindestens einen weiteren Stoff umfasst, der aus der Gruppe ausgewählt ist, bestehend aus Chitosan, Alginat, Agarose, Methylcellulose, Hyaluronan, Kollagen, Laminin, Matrigel, Fibronektin, Vitronektin, Poly-1-Lysin, Proteoglykanen, Fibrinkleber, Gelen, die durch Dezellularisierung von künstlichem und natürlichem Gewebe hergestellt werden, und Kombinationen davon.

5. Hydrogel nach einem vorstehenden Anspruch, wobei das Hydrogel mindestens ein Polymer umfasst, das aus der Gruppe ausgewählt ist, bestehend aus Polyglykolsäure (PGA), Polymilchsäure (PLA), Polycaprolacton (PCL), Polyvinylalkohol (PVA), Polyethylenglykol (PEG), Methylmethacrylat, Poly(methylmethacrylat) (PMMA), Poly(2-hydroxyethylmethacrylat) (PolyHEMA), Poly(glycerinsebacat), Polyurethanen, Poly(isopropylacrylamid), Poly(N-isopropylacrylamid) und Kombinationen davon.

6. Hydrogel nach einem vorstehenden Anspruch, wobei das Hydrogel einen pH-Wert im Bereich von etwa 6,5 bis etwa 7,5 aufweist.

7. Hydrogel nach einem vorstehenden Anspruch, wobei die Hauthaftung des Hydrogels mindestens etwa 120 g/Inch beträgt.

8. Hydrogel nach einem vorstehenden Anspruch, wobei das Hydrogel eine Dicke im Bereich von etwa 10 mil bis etwa 50 mil aufweist.

9. Hydrogel nach einem vorstehenden Anspruch, wobei das Hydrogel eine Haltbarkeit von mindestens etwa sechs Monaten aufweist.

10. Hydrogel nach einem vorstehenden Anspruch, wobei das mindestens eine lokale Betäubungsmittel mindestens einen Stoff umfasst, der aus der Gruppe ausgewählt ist, bestehend aus *Botulinumtoxin*, Saxitoxin, Tetrodotoxin, Lidocain, Bupivacain, Ropivacain, Benzocain, Pramoxin, Prilocain, Proparacain, Dibucain, Tetracain, Lidocain und Kombinationen davon.

11. Hydrogel nach einem vorstehenden Anspruch, wobei das mindestens eine Mittel gegen Hyperhidrose mindestens ein *Botulinumtoxin* und Kombinationen davon umfasst.

12. Hydrogel nach Anspruch 10 oder Anspruch 11, wobei das *Botulinumtoxin Botulinumtoxin*-Komplex A ist.

13. Wandlerarray zur Erzeugung eines elektrischen Wechselfelds, umfassend:
mindestens zwei isolierte Elektroden, wobei jede der Elektroden ein elektrisches Wechselfeld erzeugt, das eine Frequenz im Bereich von etwa 50 kHz bis etwa 10 MHz aufwest; und
das Hydrogel nach einem vorstehenden Anspruch.

## Revendications

1. Hydrogel, comprenant :
un hydrogel conducteur polymérisé destiné à être appliqué sur la peau d'un patient et à être placé entre la peau du patient et au moins un réseau de transducteurs générant un champ électrique alternatif dont la fréquence se situe entre environ 50 kHz et environ 10 MHz, dans lequel l'hydrogel conducteur polymérisé comprend au moins une composition disposée à l'intérieur ou fixée à celui-ci, dans lequel l'au moins une composition comprend au moins un agent actif choisi parmi au moins un agent anesthésiant local, au moins un agent anti-hyperhidrose, ou une combinaison de ceux-ci ; dans lequel
l'au moins une composition est formulée pour une libération contrôlée ou prolongée et comprend au moins un système d'administration pour l'administration de l'au moins un agent actif, dans lequel le système d'administration est choisi dans le groupe constitué de liposomes, de particules à base de lipides, de micro-billes, de microparticules, de nanoparticules, de conjugués polymères, d'implants de collagène et de combinaisons de ceux-ci.

2. Hydrogel selon la revendication 1, dans lequel l'hydrogel est stérile.

3. Hydrogel selon la revendication 1 ou la revendication 2, dans lequel l'hydrogel est un gel d'acide polyacrylique, un gel de povidone ou un gel de cellulose.

4. Hydrogel selon une quelconque revendication précédente, dans lequel l'hydrogel comprend au moins une substance supplémentaire choisie dans le groupe constitué de chitosane, d'alginate, d'agarose, de méthylcellulose, d'hyaluronane, de collagène, de laminine, de matrigel, de fibronectine, de vitronectine, de poly-1-lysine, de protéoglycanes, de colle de fibrine, de gels obtenus par décellularisation de tissus artificiels et naturels, et de combinaisons de ceux-ci.

5. Hydrogel selon une quelconque revendication précédente, dans lequel l'hydrogel comprend au moins un polymère choisi dans le groupe constitué de l'acide polyglycolique (PGA), de l'acide polylactique (PLA), de la polycaprolactone (PCL), de l'alcool polyvinylique (PVA), du polyéthylène glycol (PEG), du méthacrylate de méthyle, du poly(méthacrylate de méthyle) (PMMA), du poly(méthacrylate de 2-hydroxyéthyle) (PolyHEMA), du poly(sébacate de glycérol), de polyuréthanes, du poly(isopropylacrylamide), du poly(N-isopropylacrylamide) et des combinaisons de ceux-ci.

6. Hydrogel selon une quelconque revendication précédente, dans lequel l'hydrogel présente un pH compris entre environ 6,5 et environ 7,5.

7. Hydrogel selon une quelconque revendication précédente, dans lequel l'adhérence cutanée de l'hydrogel est d'au moins environ 120 g/pouce.

8. Hydrogel selon une quelconque revendication précédente, dans lequel l'hydrogel présente une épaisseur comprise entre environ 10 mil et environ 50 mil.

9. Hydrogel selon une quelconque revendication précédente, dans lequel l'hydrogel présente une durée de conservation d'au moins environ six mois.

10. Hydrogel selon une quelconque revendication précédente, dans lequel le au moins un agent anesthésiant localisé comprend au moins une substance choisie parmi le groupe constitué d'une toxine *botulique*, de saxitoxine, de tétrodotoxine, de lidocaïne, de bupivacaïne, de ropivacaïne, de benzocaïne, de pramoxine, de prilocaïne, de proparacaïne, de dibucaïne, de tétracaïne, de lidocaïne et de combinaisons de celles-ci.

11. Hydrogel selon une quelconque revendication précédente, dans lequel le au moins un agent anti-hyperhidrose comprend au moins une toxine *botulique*, et des combinaisons de celles-ci.

12. Hydrogel selon la revendication 10 ou la revendication 11 dans lequel la toxine *botulique* est le complexe de toxine *botulique* A.

13. Réseau de transducteurs pour système de génération de champ électrique alternatif, comprenant :
au moins deux électrodes isolées, dans lequel chacune des électrodes génère un champ électrique alternatif dont la fréquence se situe entre environ 50 kHz et environ 10 MHz ; et
l'hydrogel selon une quelconque revendication précédente.
